# EUROPEAN PATENT SPECIFICATION

(11) **EP 1 991 180 B1**
(45) Date of publication and mention of the grant of the patent: **05.09.2012**
(21) Application number: 07752798.4
(22) Date of filing: 09.03.2007
(51) Int. Cl.: A61F 2/68, A61H 3/00

(54) **POWER GENERATING LEG**
ENERGIEERZEUGENDES BEIN
JAMBE GENERATRICE D'ENERGIE

(30) Priority: 09.03.2006 US 781569 P
(43) Date of publication of application: 19.11.2008
(73) Proprietor: The Regents of the University of California, Oakland, CA 94607-5200 (US)
(72) Inventor: KAZEROONI, Homayoon, Berkeley, CA 94705 (US); ZOSS, Adam, Berkeley, CA 94708 (US); HARDING, Nathan, H., Orkland, CA 94618 (US); ANGOLD, Russdon, American Canyon, CA 94503 (US)
(74) Representative: Price, Nigel John King
(86) International application number: PCT/US2007/006122
(87) International publication number: WO 2007/103579

(56) References cited:
- WO-A1-2007/016781
- WO-A2-2006/113520
- WO-A2-2007/025116
- US-A- 5 961 476
- US-A1- 2004 102 723
- US-A1- 2005 167 178
- US-A1- 2005 167 178
- US-A1- 2006 046 907
- US-A1- 2006 046 907
- US-B1- 6 500 210
- US-B2- 6 676 707

## Description

### BACKGROUND

### 1. Field

The present application relates generally to the field of power generating legs.

### 2. Related Art

A human walks in a cyclic motion. Opportunities exist, therefore, to provide a device that converts walking mechanical power into electrical power. In particular this application describes a power generating leg that is configured to be coupled to a person's lower limb and generate power as the person walks. The power generating leg described here can be an orthotic leg or a prosthetic leg. In some embodiments, the power generating leg is a leg of a robotic exoskeleton.
US 2004/102723 (A1) discloses an apparatus for controlling joint movement and reducing muscle stress. The apparatus comprises: a first fastening means; a second fastening means; a stationary portion coupled to the first fastening means; a moving portion coupled to the second fastening means, the stationary and moving portions being attachable proximate to a joint of the human body with the first and second fastening means, respectively, and participating in movements of the joint; detection means operative to detect joint movements and muscle stress; an actuator operative, when energized, to exert force between the stationary and moving portions; and control means responsive to the detection means for controlling the energizing and de-energizing of the actuator, wherein the energizing is controllable for directing the force so that, when assisting, the force reduces the muscle stress and, when resisting, the force opposes joint movement. The apparatus discloses, in addition to a power utilisation mode, a power regeneration mode to return electrical power to a power supply when the apparatus is in a resisting mode.
US 2006/046907 (A1) discloses a device for generating power from a locomotion energy associated with leg muscles acting across an ankle joint. The device comprises: a cuff worn on the leg; a shoe; an energy absorbing element operatively connected to the cuff and shoe for absorbing energy during one or more periods of a periodic motion of the joint in which energy is absorbed by the muscles; and means for at least partially returning the absorbed energy to one of an energy storage device or power consuming device.
US 2005/167178 (A1) discloses a road vehicle utilizing hydraulic machines for primary and accessory vehicle drive functions. An internal combustion engine directly drives a first variable capacity hydraulic machine selectively operable as pump or motor, an electric machine electrically coupled to a storage battery is selectively operable as motor or generator and is mechanically coupled to a second hydraulic machine operable as pump or motor, and at least one hydraulic drive motor is coupled to a vehicle drive wheel. A controller regulates the capacity of the first hydraulic machine, and controls the functionality of the first and second hydraulic machines to selectively crank the engine or charge the storage battery. The first hydraulic machine also supplies hydraulic fluid to accessory drive motors including a power steering drive motor, an air compressor drive motor and a supercharger drive motor.
WO 2007/016781 (A1) was published on 15 February 2007 and is admissible as prior art only under Article 54(3) EPC. It discloses an apparatus for harvesting energy from motion of one or more joints. The apparatus comprises: a generator for converting mechanical energy into corresponding electrical energy; one or more sensors for sensing one or more corresponding characteristics associated with motion of the one or more joints; and control circuitry connected to receive the one or more sensed characteristics and configured to assess, based at least in part on the one or more sensed characteristics, whether motion of the one or more joints is associated with mutualistic conditions or non-mutualistic conditions. If the control circuitry determines that the motion of the one or more joints is associated with particular mutualistic conditions, the control circuitry is configured to engage energy harvesting by completing a coupling of mechanical energy associated with the motion of the one or more joints to the generator and electrical output of the generator to a load. If the control circuitry determines that the motion of the one or more joints is associated with non-mutualistic conditions, the control circuitry is configured to disengage energy harvesting by decoupling at least one of: the mechanical energy associated with the motion of the one or more joints from the generator and the electrical output of the generator from the load.
WO 2007/025116 (A2) was published on 1 March 2007 and is admissible as prior art only under Article 54(3) EPC. It discloses a prosthetic leg having an electronically controlled regenerative prosthetic knee. The knee may be passive, whereby it is used only to generate electrical energy. In other embodiments, the knee may be active, whereby it can be used to assist with or completely control gait, as well as to generate electrical energy. The knee makes use of an actuator motor/generator to control gait and/or to generate electrical energy. An electronic control system is provided to control overall operation of the prosthetic leg, to distribute generated electrical energy, and to transfer excess electrical energy to one or more storage devices for later use.
WO 2006/113520 A2, which is regarded as closest prior art, was published on 26 October 2006 and is, at most, admissible as prior art only under Article 54(3)EPC. It discloses a lower extremity exoskeleton comprising two leg supports connectable to person's lower limbs and configured to rest on the ground during their stance phase. Each leg support comprises a thigh link and a shank link and a knee joint configured to allow flexion and extension between the shank link and the thigh link. The lower extremity exoskeleton further comprises an exoskeleton trunk connectable to the person's upper body. The exoskeleton trunk is connectable to the thigh links of the leg supports allowing for the flexion and extension between the leg supports and the exoskeleton trunk. Two hydraulic torque generators are coupled to each of the knee joints. A power unit, capable of providing power, is coupled to the torque generators.
The power unit includes a first hydraulic path between a fluid reservoir and each of the hydraulic torque generators, said first hydraulic path comprising a hydraulic pump connected in series with a pump isolating valve, said hydraulic pump being rotatably coupled to an electric motor, and a second hydraulic path between the fluid reservoir and said hydraulic torque generator including an actuated flow restricting valve. In operation, when a leg support is in a stance phase and climbing a slope or stairs, the power unit injects power into the respective torque generator by means of the hydraulic pump, the corresponding actuated flow restricting valve being shut and the corresponding pump isolating valve being open, thereby extending the respective knee angle. When a leg support is in stance phase and not climbing a slope or stairs, the power unit does not inject any power to the respective torque generator, but forces the torque generator to resist flexion of the respective knee joint, the corresponding pump isolating valve disconnecting the fluid flow between the hydraulic pump and the torque generator while the actuated flow restricting valve restricts the fluid flow from the torque generator. When a leg support is in a swing phase, the power unit does not inject any power to the respective torque generator, but without dissipating any stored power in said power unit, it forces the torque generator to minimize its resistance to knee flexion and extension. There is a disclosure of energy capture when walking down a slope or stairs, the corresponding pump isolating valve being-opened and the actuated flow restricting valve being closed such that the hydraulic pump behaves as a hydraulic motor turning the electric motor backwards and generating electricity. There is also a disclosure of dissipating energy through a device, such as a flow restricting valve, during stance while walking on near level ground.

### SUMMARY

According to a first aspect of the present invention there is provided the power generating leg of claim 1.

According to a second aspect of the present invention there is provided the power generating leg operating method of claim 14.

Additional features of the power generating leg and method are set out in the dependent claims.

### DRAWINGS

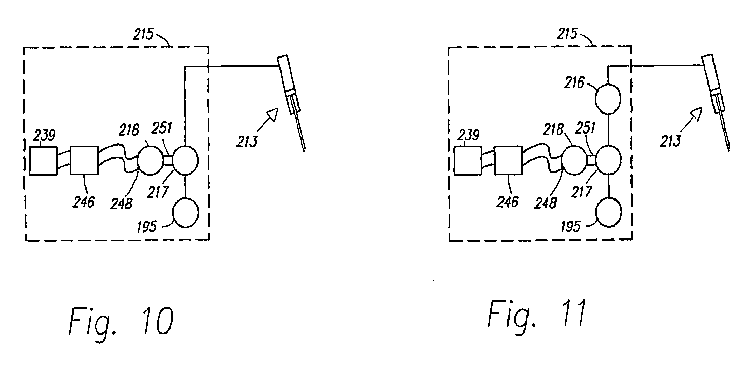

Embodiments of power generating leg and methods of operation thereof in accordance with the present invention will now be described, by way of example only, with reference to the accompanying drawings. Although the power generating leg power units of Figs. 10 and 11 are not in accordance with the invention as claimed, they represent technical background that is useful for understanding the power generating leg of the present invention. In the accompanying drawings like characters represent like parts. The drawings are described below.

Fig. 1 is a side view of an exemplary embodiment of a power generating leg.

Fig. 2 is a side view of an exemplary embodiment of a power generating leg coupled to a person's lower limb.

Fig. 3 is a side view of an exemplary embodiment of a power generating leg coupled to a person's lower limb.

Fig. 4 is a perspective view of an exemplary embodiment of a power generating leg.

Fig. 5 is a side view of an exemplary embodiment of a power generating leg coupled to a person's lower limb that acts as a prosthetic leg.

Fig. 6 is a side view of an exemplary embodiment of a power generating leg coupled to a person's lower limb.

Fig. 7 is a side view of an exemplary embodiment of a power generating leg coupled to a person's lower limb.

Fig. 8 is a side view of an exemplary embodiment of a power generating leg coupled to a person's lower limb.

Fig. 9 is a side view of a portion of an exemplary embodiment of a power generating leg.

Fig. 10 is a block diagram of a power unit.

Fig. 11 is a block diagram of a power unit.

Fig. 12 is a block diagram of an exemplary embodiment of a power unit according to the invention.

Fig. 13 is a block diagram of an exemplary embodiment of a power unit according to the invention.

Fig. 14 is a graph depicting the knee power and the knee angle where a human is descending steps.

Fig. 15 is a graph depicting the knee torque and the knee angle where a human is walking on a level ground.

Fig. 16 shows the knee power and the knee angle where a human is ascending steps.

Fig. 17 is a block diagram of an exemplary embodiment of a power unit.

Fig. 18 is a block diagram of an exemplary embodiment of a power unit.

### DETAILED DESCRIPTION

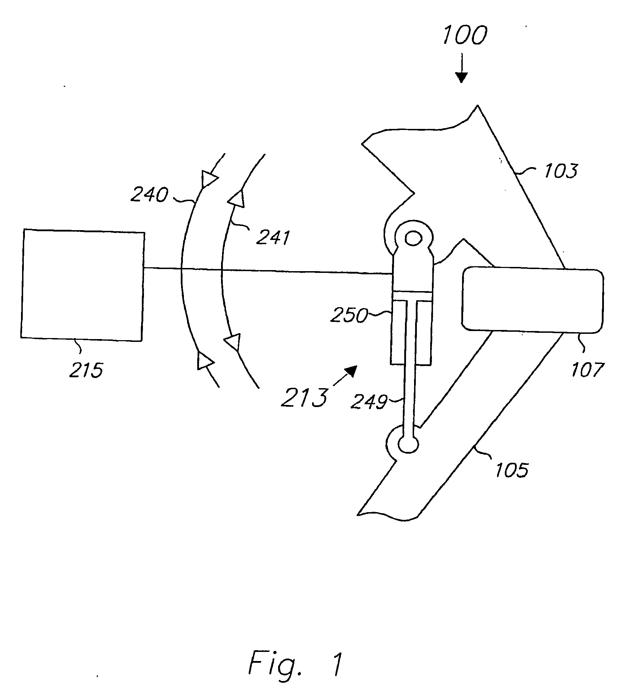

In accordance with an embodiment, **Fig. 1** is a drawing illustrating a power generating leg **100.** Power generating leg **100** is configured to be coupled to a person's lower limb. Power generating leg **100** comprises a thigh link **103** and a shank link **105.** A knee mechanism **107** is connected to thigh link **103** and shank link **105.** Knee mechanism **107** is configured to allow flexion movements (in the direction of arrows **240)** and extension movements (in the direction of arrows **241)** between thigh link **103** and shank link **105.** A torque generator **213** is configured to impose a torque that affects the movements of thigh link **103** and shank link **105** relative to each other. A power unit **215** is coupled to torque generator **213,** and is configured to cause torque generator **213** to generate torque.

When power unit **215** operates in a power regeneration mode, power unit **215** is configured to cause torque generator **213** to generate a torque that opposes the angular velocity of thigh link **103** and shank link **105** relative to each other. In this power regeneration mode, power unit **215** further converts a portion of the power associated with the product of the generated torque and the angular velocity of shank link **105** and thigh link **103** relative to each other into electrical power to be stored in a storage device. In some embodiments, the storage device is located within power unit **215.** In some embodiments, the storage device is external. In some embodiments, the converted electrical power is stored in a set of batteries. In some embodiments, the converted electrical power is stored in a set of capacitors.

In some embodiments, power unit **215** further operates in a power utilization mode. During this mode, power unit **215,** using stored power, is configured to cause torque generator **213** to generate a torque. In some embodiments, a portion of the power used in the power utilization mode is the electrical power generated in the power regeneration mode.

In some embodiments, power unit **215** further operates in a power dissipation mode. During this mode, power unit **215** is configured to cause torque generator **213** to generate a torque that opposes the angular velocity of thigh link **103** and shank link **105** relative to each other. Power unit **215** also dissipates the power associated with the product of the torque and the angular velocity of thigh link **103** and shank link **105** relative to each other.

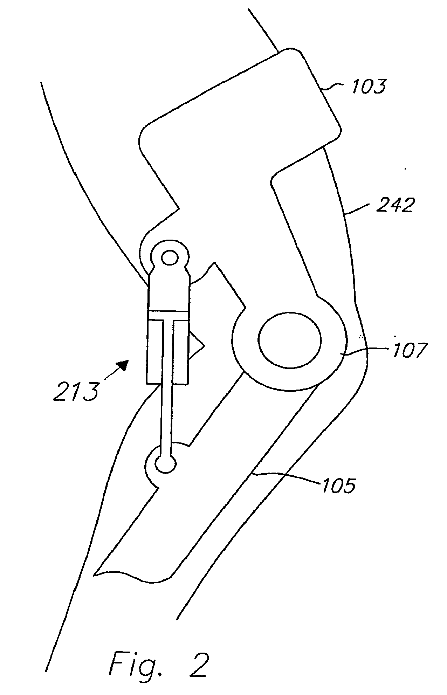

In some embodiments, as shown in **Fig. 2****,** knee mechanism **107** comprises at least one rotary joint. The rotary joint is configured to allow for rotary flexion and extension movements of thigh link **103** and shank link **105** relative to each other.

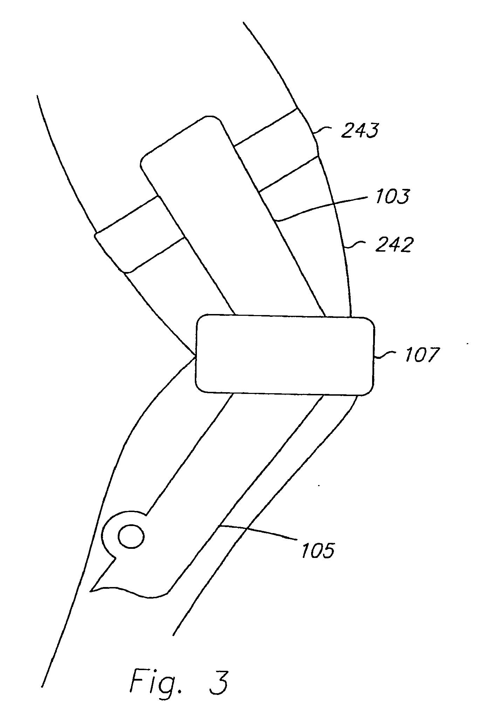

In some embodiments, as shown in **Fig. 2****,** thigh link **103** is in contact with a person's thigh **242** so it can be moved by the person's thigh. In some embodiments, thigh link **103** is connectable to a person's thigh. **Fig. 3** shows an embodiment where power generating leg **100** comprises a thigh strap **243** connectable to a person's thigh **242** and causes thigh link **103** to move when the person's thigh moves. Torque generator **213** is not shown in **Fig. 3** for clarity.

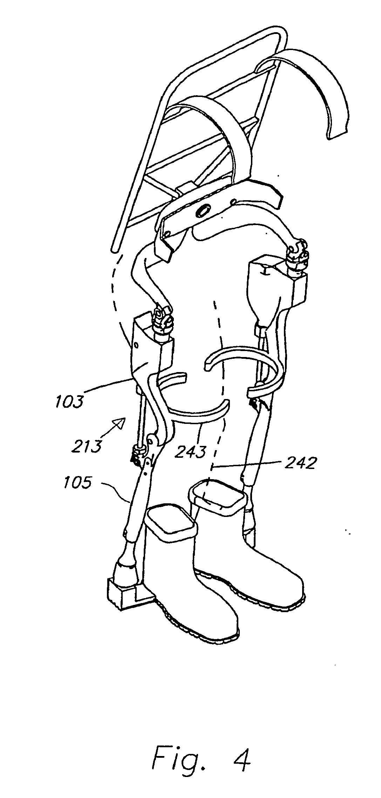

In some embodiments, thigh strap **243,** among other components, comprises compliant materials wrapped around the person's thigh. Compliant materials comprise an element or combination of elements selected from a group consisting of fabric, textile, elastomer, rubber and velcro materials. In some embodiments, as shown in **Fig. 4****,** thigh strap **243** comprises a bracket formed to partially embrace the person thigh.

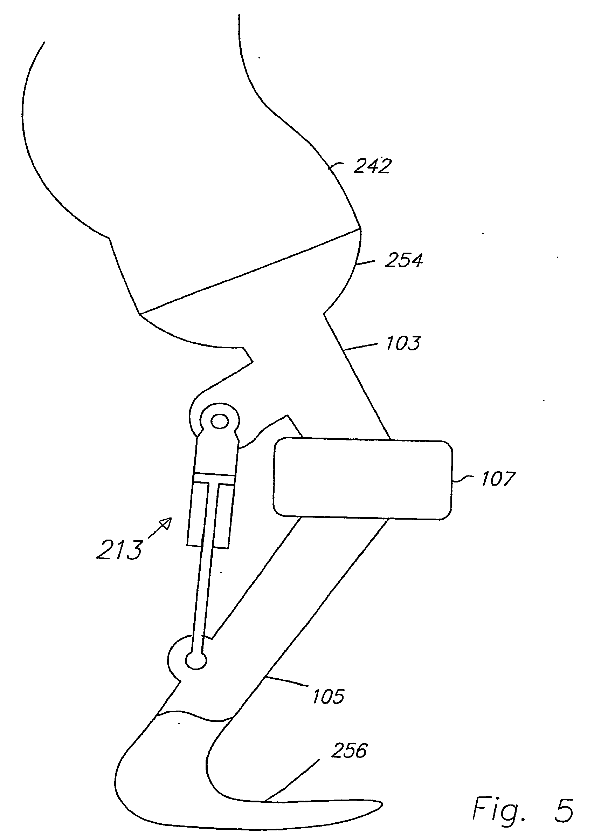

In some embodiments, as shown in **Fig. 5****,** power generating leg **100** acts as a prosthetic leg for above-knee amputees. In this case, thigh link **103** comprises a socket **254** attachable to an amputee's remaining thigh. Therefore, power generating leg **100** functions as an above knee prosthetic device. In some embodiments, power generating leg **100** includes an artificial foot **256.**

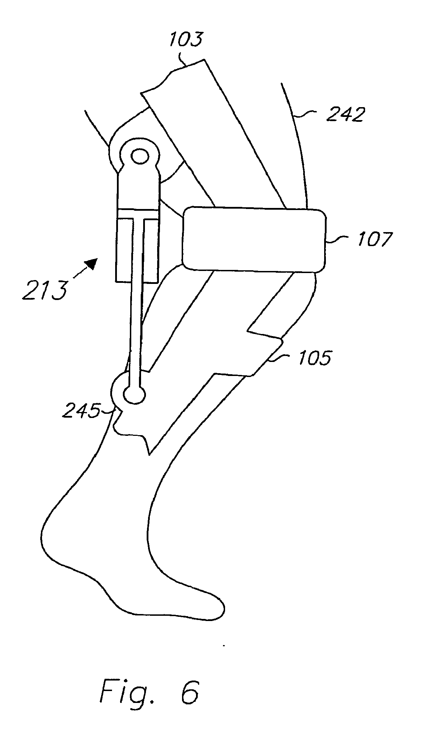

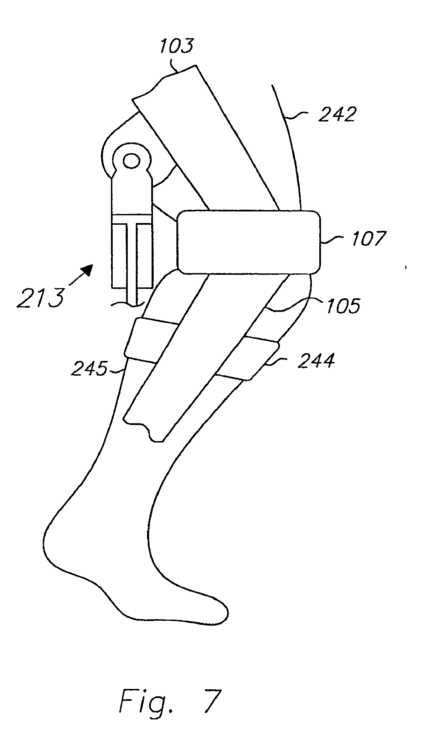

According to an embodiment shown in **Fig. 6****,** shank link **105** is configured to be in contact with a person's shank so it can be moved by the person's shank. In some embodiments, shank link **105** is configured to be connected to a person's shank. **Fig. 7** shows an embodiment where power generating leg **100** comprises a shank strap **244** configured to be connected to a person's shank **245** and causes shank link **105** to move when the person's shank moves. Torque generator **213** is partially shown in **Fig. 7** for clarity.

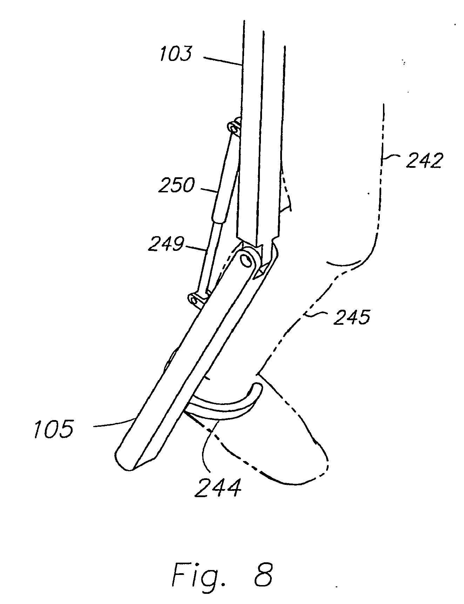

In some embodiments, shank strap **244,** among other components, comprises compliant materials wrapped around the person's shank **245.** Compliant materials comprise an element or combination of elements selected from a group consisting of fabric, textile, elastomer, rubber and velcro materials. In some embodiments, as shown in **Fig. 8****,** shank strap **244** comprises a bracket formed to partially embrace the person's shank **245.**

In summary, power generating leg **100** can be an orthotic leg or a prosthetic leg. In some embodiments, power generating leg **100** is a leg of an exoskeleton as shown in **Fig. 4****.**

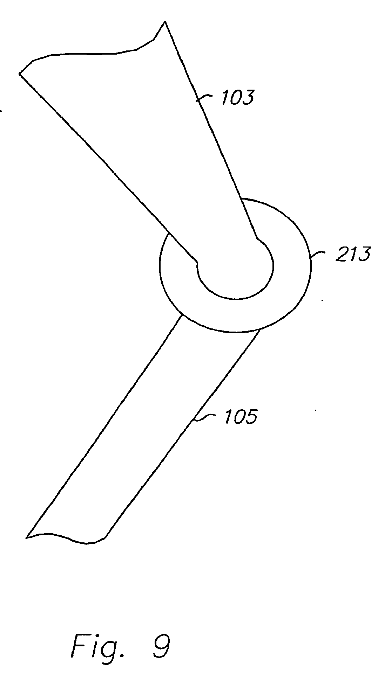

In some embodiments, as shown in **Fig. 1****,** torque generator **213** comprises a hydraulic torque generator where a pressurized hydraulic fluid, by pushing against moving surfaces, generates torque. Examples of hydraulic torque generators **213** include, without limitation, linear hydraulic piston-cylinders and rotary hydraulic actuators where pressurized hydraulic fluid, by pushing against moving surfaces, generate force or torque. An illustration of a power generating leg **100** utilizing linear hydraulic piston-cylinder torque generator **213** is shown in **Fig. 1****,** where piston **249** slides relative to cylinder **250.** An illustration of a power generating device **100** utilizing a rotary hydraulic actuator is shown in **Fig. 9****.** In some embodiments, as shown in **Fig. 9****,** torque generator **213** is integrated into the construction of knee mechanism **107.**

**Fig. 10** shows a power unit **215** not in accordance with the present invention. Power unit **215** comprises a hydraulic motor **217** where its two hydraulic ports are connected to torque generator **213** and a fluid reservoir **195.** Hydraulic reservoir **195** may be a separate reservoir as shown, or it may be the other cavity of the torque generating device **213.** Hydraulic motor **217** is a device that converts hydraulic fluid flow into mechanical rotation of a rotating shaft **251.**

An electric generator **218,** capable of producing electric voltage on its terminals **248,** is rotatably coupled to rotating-shaft **251** of hydraulic motor **217.** When power unit **215** is in power regeneration mode, electric generator **218** generates electric voltage on its terminals **248** when the hydraulic fluid flow between torque generator **213** and fluid reservoir **195** causes the rotor of hydraulic motor **217** to turn. The generated voltage on terminals **248** is a function of the angular velocity of thigh link **103** and shank link **105** relative to each other. When an electric current is allowed to pass through terminals **248** such that the product of current and the generated voltage indicates that electric power is generated, hydraulic motor **217** resists the fluid flow that is induced by the motion of thigh link **103** and shank link **105** relative to each other. This resistance to fluid flow causes torque generator **213** to impose a torque, which resists the motion of thigh link **103** and shank link **105** relative to each other. This torque is a function of the electric current that is flowing through terminals **248** of electric generator **218.** By controlling the electric current that is flowing through terminals **248,** one can control the torque generated by hydraulic torque generator **213.**

When power unit **215** is in power regeneration mode, power unit **215** converts only a portion of the mechanical power associated with the product of the generated torque and the relative speed of thigh link **103** and shank link **105** into electric power. The rest of this power is converted into heat in such things as the motor winding and hydraulic fluid. The power unit **215** may further comprise a battery charging unit **246** that charges at least one battery **239.** The battery **239** may be located outside power unit **215.**

When power unit **215** is operating in power utilization mode, hydraulic motor **217** acts like a hydraulic pump and electric generator **218** acts like an electric motor. In operation, when power unit **215** is in power utilization mode, hydraulic motor **217** causes torque generator **213** to generate torque when an electric current passes through terminals **248** of electric generator **218.** The generated torque in torque generator **213** is a function of the electric current passing through terminals **248.** By controlling the electric current that is passing through terminals **248,** one can control the torque generated by hydraulic torque generator **213.** When hydraulic fluid flows between fluid reservoir **195** and torque generator **213,** a portion of stored electrical power is converted into the mechanical power associated with the product of the generated torque and the relative speed of thigh link **103** and shank link **105** into electric power.

Examples of electric generator **218** include, without limitation, AC (alternating current) generators, brush-type DC (direct current) generators, brushless DC generators, electronically commutated motors (ECMs), and combinations thereof. Examples of hydraulic motor **217** include, without limitation, hydraulic gear motors, axial piston pumps, rotary piston pumps, vane type hydraulic motors and combinations thereof.

**Fig. 11** shows another power unit **215**, also not in accordance with the present invention. This power unit is similar to the power unit shown in **Fig. 10** but a motor isolating valve **216** is located in series with hydraulic motor **217.** The operation of the Fig. 11 power unit, when motor isolating valve **216** is in an open position, is similar to the operation of the power unit of **Fig. 10****.** When the motor isolating valve is in a closed position, no hydraulic flow is permitted and therefore thigh link **103** and shank link **105** cannot move relative to each other. This characteristic can be used during stance. Motor isolating valve **216** comprises any valve or combination of valves capable of performing the indicated functions. Examples of motor isolating valve **216** include, without limitation, flow control valve, pressure control valve, actuated needle valves, solenoid valves and on-off valve.

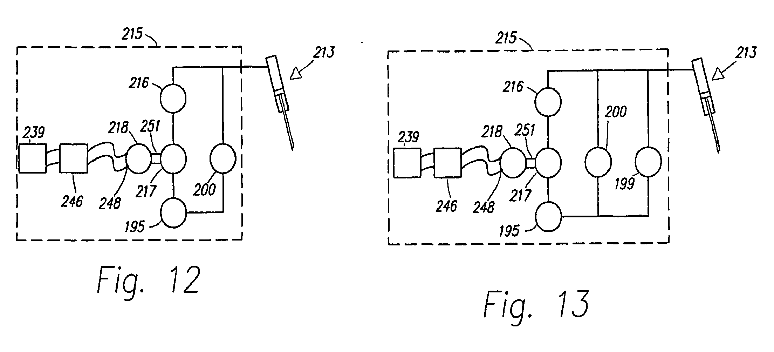

According to the invention, as shown **in** **Fig. 12****,** power unit **215** further comprises an actuated flow restricting valve **200** with adjustable orifice coupled to torque generator **213** and fluid reservoir **195** creating a second hydraulic path between torque generator **213** and fluid reservoir **195.** When motor isolating valve **216** is in a closed position, actuated flow restricting valve **200** can be adjusted to allow proper resistance for the fluid flow between torque generator **213** and fluid reservoir **195.** The amount of this resistance to fluid flow causes torque generator **213** to impose a resistive torque which resists the motion of thigh link **103** and shank link **105** relative to each other. This characteristic is used in power dissipation mode and can be used to damp the knee motion. Actuated flow restricting valve **200** comprises any valve or combination of valves capable of performing the indicated functions. Examples of actuated flow restricting valve 200 include, without limitation, flow control valve, pressure control valve, actuated needle valves, solenoid valves and on-off valve.

In some embodiments, as shown in **Fig. 13****,** power unit **215** further comprises a check valve (one-way valve) **199** coupled to torque generator **213** and fluid reservoir **195** creating another hydraulic path between torque generator **213** and fluid reservoir **195.** Check valve **199** allows for minimum resistance hydraulic flow from fluid reservoir **195** to torque generator **213** during extension movement of thigh link **103** and shank link **105** relative to each other at all times.

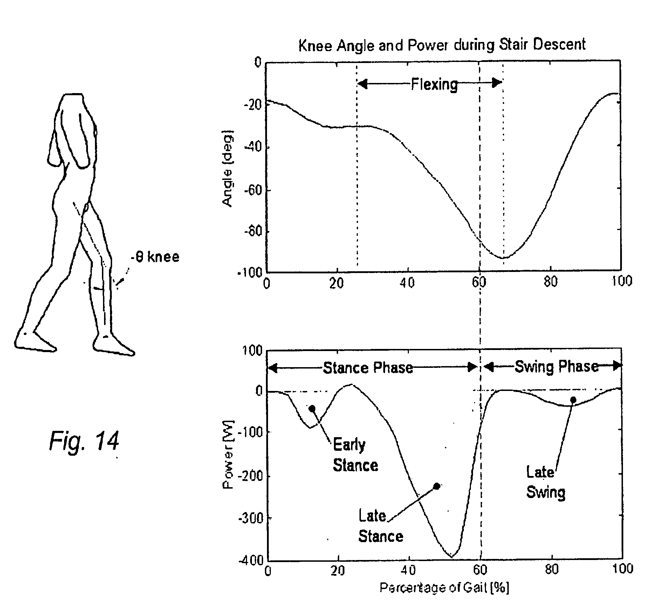

In operation, there are opportunities where power unit **215** moves into power regeneration mode and therefore power unit **215** converts a portion of the power associated with the product of the generated torque and the angular velocity of shank link **105** and thigh link **103** relative to each other into electrical power. **Fig. 14** shows the knee angle and the knee power when a human is descending steps. Negative values for power identify the regions that have potential for power regeneration. A power regeneration region is identified where power regenerating leg **100** is in a stance phase and flexing. Stance phase is defined as a configuration where power regenerating leg **100** or the human leg it is connected to is on the ground. In some embodiments, power unit **215** is configured to operate in a power regeneration mode when power generating leg **100** is in a stance phase and is descending slopes and stairs. In some embodiments, power unit **215** is configured to operate in a power regeneration mode when said power generating leg **100** is in a stance phase and is flexing. In some embodiments, power unit **215** is configured to operate in a power regeneration mode when said power generating leg **100** is in a stance phase.

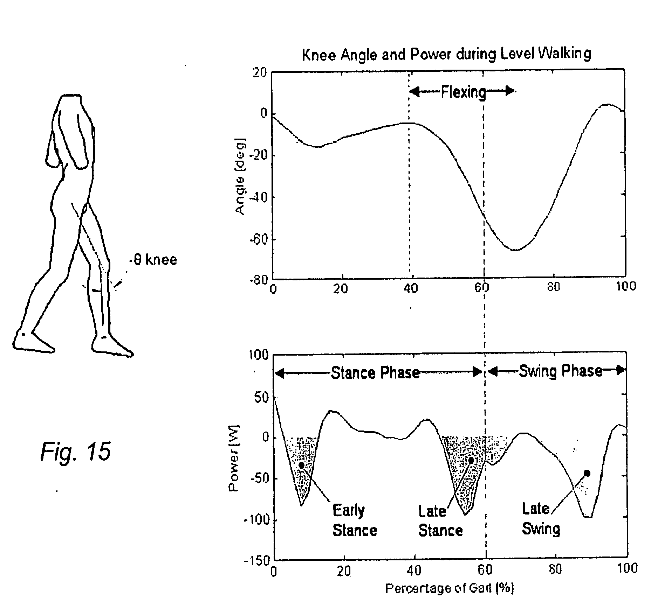

**Fig. 15** shows the knee angle and the knee power when a human is walking on level grounds. Negative values for power identify the regions that have potential for power regeneration. In some embodiments, power unit **215** is configured to operate in power regeneration mode when power generating leg **100** is in a stance phase and is walking on level grounds. In some embodiments, power generating leg **100** is configured to operate in a power regeneration mode when power generating leg **100** is in a swing phase.

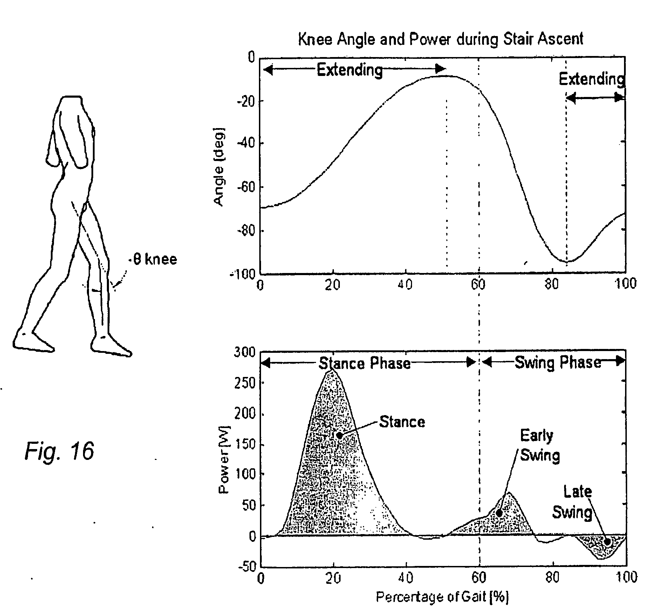

In operation, there are opportunities where power unit **215** moves into power utilization mode. **Fig. 16** shows the knee torque and the knee angle when a human is ascending steps. Positive values for power identify the regions that have potential for power utilization. In some embodiments, power unit **215** is configured to operate in power utilization mode when power generating leg **100** is in a stance phase and is ascending slopes and stairs. In some embodiments, power unit **215** is configured to operate in power utilization mode when power generating leg **100** is in a stance phase and is extending.

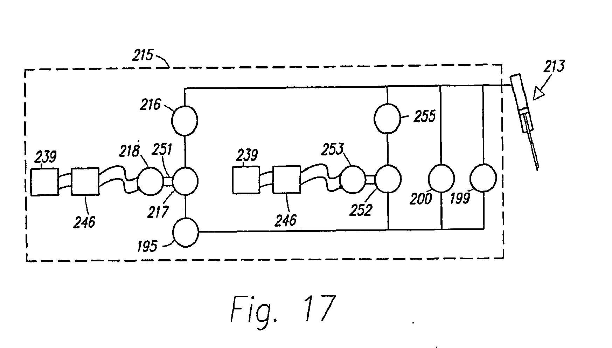

In some embodiments, power unit **215,** as shown in **Fig. 17****,** comprises two electric generators and hydraulic motors. Power unit **215** is coupled to hydraulic torque generator **213,** and among other components, comprises a first hydraulic path between torque generator **213** and fluid reservoir **195** comprising a first hydraulic motor **217.** Power unit **215** further comprises a second hydraulic path between torque generator **213** and fluid reservoir **195** comprising a second hydraulic motor **252.** A first electric generator **218** is rotatably coupled to first hydraulic motor **217.** A second electric generator **253** is rotatably coupled to second hydraulic motor **252.** In operation, when power unit **215** is in a power regeneration mode, at least one of electric generators **218** and **253** generates an electric voltage when the hydraulic fluid flows from torque generator **213** to said reservoir **195.** Motor isolating valves **216** and **255** are configured to select either hydraulic motor **217** or **252** for operation. These two isolating valves **216** and **255** can be replaced by a three way selector valve selecting between hydraulic motor **217** or **252.** Power unit **215** further comprises a battery charging unit **246** that charges at least one battery **239.**

In some embodiments, first electric generator **218** is used in a power regeneration mode when power generating leg **100** is in a stance phase and descending a stair or a slope. In this case, motor isolating valve **255** is closed and stops the fluid flow to second hydraulic motor **252** while motor isolating valve **216** is open and allows for the fluid flow to first hydraulic motor **217.** In such embodiments, first electric generator **218** is used as an electric motor in a power utilization mode when power generating leg **100** is in a stance and ascending a stair or a slope.

In some embodiments, second electric generator **253** is used in a power regeneration mode when power generating leg **100** is in a stance phase and walking on level ground. In this case, motor isolating valve **216** is closed and stops the fluid flow to first hydraulic motor **217,** while motor isolating valve **255** is open and allows for the fluid flow to second hydraulic motor **252.** In such embodiments, second electric generator **253** may be used as an electric motor in a power utilization mode when power generating leg **100** is in a swing phase.

In some embodiments, power unit **215** further comprises a third hydraulic path including a flow restricting valve **200.** In operation, flow restricting valve **200** is used to create controllable resistance to fluid flow. This characteristic is used in power dissipation mode and can be used to damp the knee motion.

In some embodiments, power unit **215** further comprises a forth hydraulic path including a one way valve **199.** In operation, one way valve **199** allows for minimum resistance flow from said reservoir **195** to torque generator **213** at all times.

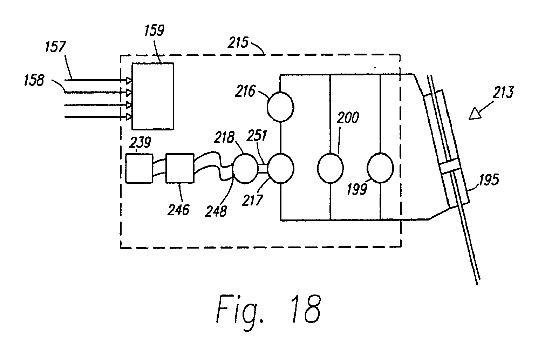

In some embodiments, as shown in **Fig. 18****,** if torque generator **213** is a rotary hydraulic actuator or a double acting, double rodded hydraulic cylinder, then hydraulic reservoir **195** may be the other cavity of torque generator **213.**

In some embodiments, power generating leg **100,** among other sensors, comprises at least one stance sensor. Stance sensor produces a stance signal **157.** Stance signal **157** identifies if power generating leg **100** is in a stance phase or in a swing phase. In some embodiments, stance signal **157** represents the magnitude of the ground reaction force to power generating leg **100.** During swing phase, stance signal **157** will detect a small or zero magnitude for ground reaction force. Stance sensor comprises an element or combination of elements selected from a group consisting of force sensor, pressure sensor, and switches capable of performing the indicated functions.

In some embodiments, power generating leg **100,** among other sensor, comprises at least one knee angle sensor. Knee angle sensor produces a knee angle signal **158.** Knee angle signal **158** identifies the angle between shank link **105** and thigh link **103.** Knee angle sensor comprises an element or combination of elements selected from a group consisting of encoder; revolver, potentiometer; LVDT, and inclinometer capable of performing the indicated functions.

In some embodiments, as shown in **Fig. 18****,** power unit **215** further comprises a signal processor **159** is configured to generate command signals for various components of power unit **215** to control power unit **215.** In some embodiments, signal processor **159** receives stance signal **157.** In some embodiments, signal processor **159** receives knee angle signal **158.** Signal processor **159** comprises an element or combination of elements selected from a group consisting of analog devices; analog compulation modules; digital devices including, without limitation, small-, medium-, and large-scale integrated circuits, application specific integrated circuits, programmable gate arrays, and programmable logic arrays; and digital computation modules including, without limitation, microcomputers, microprocessors, microcontrollers, and programmable logic controllers. In some embodiments, signal processor **159** comprises an element or combination of elements selected from a group consisting of electromechanical relays or MOSFET switches. Signal processor **159** may be located inside or outside of power unit **215.**

There are many control algorithms by which signal processor **159** could control power unit **215.** In some embodiments, when power generating leg **100** is descending a slope or stairs and is in a stance phase, signal processor **159** generates command signals so power unit **215** goes into power regeneration mode. A knee angle near 180° at the beginning of stance and a smaller angle at the end of stance may represent the situation where power generating leg **100** is descending a slope or stairs. See, U.S. Patent Application Ser. No. 10/976,652, titled LOWER EXTREMITY ENHANCER, filed on October 29, 2004; U.S. Patent Application Ser. No. 11/335,392, titled LOWER EXTREMITY EXOSKELETON, filed on January 18, 2006; and U.S. Patent Application Ser. No. 11/404,719, titled SEMI-POWERED LOWER EXTREMITY EXOSKELETON, filed on April 13, 2006.

In some embodiments, when power generating leg **100** is ascending a slope or stairs and is in a stance phase, signal processor **159** generates command signals so power unit **215** operates in the power utilization mode. This mode aids the wearer in climbing slopes and stairs. A small knee angle at the beginning of stance phase may represent the situation where power generating leg **100** is ascending a slope or stairs.

In some embodiments, when power generating leg **100** is walking on level ground and is in stance phase, signal processor generates command signals so power unit **215** operates in the power dissipation mode or power regeneration mode depending on operator preference. A large knee angle close to 180° degrees at the beginning of stance phase which does not change for a long period of stance may represent the situation where power generating leg **100** is walking on level ground.

In some embodiments, when power generating leg **100** is in a swing phase, signal processor **159** generates command signals so power unit **215** goes into power dissipation mode or power utilization mode to assist the swinging depending on operator preference.

Although the foregoing invention has been described in some detail for purposes of clarity of understanding, it will be apparent that certain changes and modifications may be practiced within the scope of the appended claims. Therefore, the described embodiments should be taken as illustrative and not restrictive, and the invention should not be limited to the details given herein but should be defined by the following claims and their full scope of equivalents.

## Claims

1. A power generating leg (10), configured to be coupled to a person's lower limb, comprising:
a thigh link (103);
a shank link (105);
a knee mechanism (107) connected to said thigh link and said shank link, and configured to allow flexion and extension movements of said thigh link and said shank link relative to each other;
a hydraulic torque generator (213) configured to generate torque between said shank link and said thigh link; and
a power unit (215) coupled to said torque generator, configured to cause said torque generator to generate torque and configured to operate in a power regeneration mode, a power utilization mode and a power dissipating mode, said power unit including a first hydraulic path between a fluid reservoir (195) and the hydraulic torque generator, said first hydraulic path comprising a hydraulic motor (217) connected in series with a motor isolating valve (216), said hydraulic motor being rotatably coupled to an electric generator (218), and a second hydraulic path between the fluid reservoir and the hydraulic torque generator including an actuated flow restricting valve (200);
wherein when said power unit is in the power regeneration mode, said power unit causes said torque generator to generate a torque that opposes the angular velocity of said thigh link and said shank link relative to each other and said power unit converts a portion of the power associated with the product of said torque and said angular velocity of said shank link and thigh link relative to each other into electrical power, and wherein said power unit is configured to operate in the power regeneration mode when the power generating leg is walking on level ground;
wherein when said power unit is in the power regeneration mode, said motor isolating valve is in an open position and said hydraulic motor resists the hydraulic flow induced by the motion of said hydraulic torque generator and an electric current passes through the terminals of said electric generator indicating that electric power is being generated by said electric generator;
wherein when said power unit is in the power utilization mode, said motor isolating valve is in an open position and said hydraulic motor is powered by the electric generator and actuates the hydraulic torque generator to create a torque between the thigh link and the shank link; and
wherein when said power unit is in the power dissipation mode, said motor isolating valve is in a closed position and said actuated flow restricting valve restricts fluid flow between the hydraulic torque generator and the fluid reservoir and causes the hydraulic torque generator to resist motion of the thigh link and the shank link with respect to one another.

2. The power generating leg of claim 1, wherein when said power unit (215) is in the power utilization mode, said power unit, using stored electrical power, causes said torque generator (213) to generate a torque.

3. The power generating leg of claim 2, wherein said power unit is configured to operate in the power utilization mode when said power generating leg is in a stance phase and is ascending slopes and stairs.

4. The power generating leg of claim 1, wherein said thigh link (103) is configurable to be connected to a person's thigh.

5. The power generating leg of claim 1, wherein said shank link (105) is configurable to be connected to a person's shank.

6. The power generating leg of claim 1, wherein said thigh link (103) comprises a socket (254) configurable to be attached to an amputee's remaining thigh and therefore said power generating leg functions as an above knee prosthetic device.

7. The power generating leg of claim 1, wherein said power unit (215) is configured to operate in the power regeneration mode either:
when said power generating leg is in a stance phase; or
when said power generating leg is in a stance phase and is flexing; or
when said power generating leg is in a swing phase.

8. The power generating leg of claim 1, wherein said power unit (215) is configured to operate in the power utilization mode when said power generating leg is in a stance phase and is extending.

9. The power generating leg of claim 5, wherein said power generating leg further comprises an artificial foot (256) coupled to said shank link (105).

10. The power generating leg of claim 1, wherein said power unit is configured to operate in the power regeneration mode when said power generating leg is either a) in a stance phase and is flexing, or b) in a stance phase and is descending slopes and stairs.

11. The power generating leg of claim 1, where said power unit (215) further comprises a one-way valve (199) coupled to said torque generator (213) and said fluid reservoir (195) creating a third hydraulic path between said hydraulic torque generator and said fluid reservoir providing minimum resistance hydraulic flow from said fluid reservoir to said torque generator at all times.

12. The power generating leg of claim 1, further comprising a third hydraulic path including a second electric generator (253) rotatably coupled to a second hydraulic motor (252);
wherein when said power unit (215) is in said power regeneration mode, at least one of said first or second hydraulic motors (217, 252) resists the hydraulic flow induced by the motion of said hydraulic torque generator (213) and an electric current passes through the terminals of the corresponding first or second electric generator such that electric power is generated by said first or second electric generator.

13. The power generating leg of claim 12, wherein the torque generator (213) is coupled to the knee mechanism; said first electric generator (218) is used as an electric motor in the power utilization mode when said power generating leg is in a stance phase and ascending a stair or slope; said first electric generator is used in the power regeneration mode when said power generating leg is in a stance phase and descending a stair or slope and is used as an electric motor in the power utilization mode when said power generating leg is in a stance phase and ascending a stair or slope; and said second electric generator (253) is used in the power regeneration mode when said power generating leg is walking on level ground.

14. A method of operating a power generating leg (10) configured to be coupled to a person's lower limb and including: a thigh link (103); a shank link (105); a knee mechanism (107) connected to said thigh link and said shank link, and configured to allow flexion and extension movements of said thigh link and said shank link relative to each other; a hydraulic torque generator (213) configured to generate torque between said shank link and said thigh link; and a power unit (215) coupled to said torque generator, configured to cause said torque generator to generate torque and configured to operate in a power regeneration mode, a power utilization mode and a power dissipating mode, said power unit including a first hydraulic path between a fluid reservoir (195) and the hydraulic torque generator, said first hydraulic path comprising a hydraulic motor (217) connected in series with a motor isolating valve (216), said hydraulic motor being rotatably coupled to an electric generator (218), and a second hydraluic path between the fluid reservoir and the hydraulic torque generator including an actuated flow restricting valve (200), said method comprising:
when said power unit is in the power regeneration mode, said power unit causing said torque generator to generate a torque that opposes the angular velocity of said thigh link and said shank link relative to each other and said power unit converting a portion of the power associated with the product of said torque and said angular velocity of said shank link and thigh link relative to each other into electrical power, said power unit being operable in the power regeneration mode when the power generating leg is walking on level ground;
when said power unit is in the power utilization mode, said motor isolating valve being in an open position and said hydraulic motor being powered by the electric generator and actuating the hydraulic torque generator to create a torque between the thigh link and the shank link; and
when said power unit is in the power dissipation mode, said motor isolating valve being in a closed position and said actuated flow restricting valve restricting fluid flow between the hydraulic torque generator and the fluid reservoir and causing the hydraulic torque generator to resist motion of the thigh link and the shank link with respect to one another; and
wherein when said power unit is in the power regeneration mode, said motor isolating valve is in an open position and said hydraulic motor resists the hydraulic flow induced by the motion of said hydraulic torque generator and an electric current passes through the terminals of said electric generator indicating that electric power is being generated by said electric generator.

15. The method of claim 14, wherein said power unit is also operable in the regeneration mode:
when said power generating leg is in a stance phase; or
when said power generating leg is in a stance phase and is flexing; or
when said power generating leg is in a swing phase.

## Patentansprüche

1. Leistung erzeugendes Bein (10), welches ausgestaltet ist, um mit einem Unterschenkel einer Person gekoppelt zu werden, umfassend:
eine Oberschenkelverbindung (103);
eine Unterschenkelverbindung (105);
einen Kniemechanismus (107), welcher mit der Oberschenkelverbindung und der Unterschenkverbindung verbunden ist und welcher ausgestaltet ist, um beugende und streckende Bewegungen der Oberschenkelverbindung und der Unterschenkelverbindung relativ zueinander zu ermöglichen;
einen hydraulischen Drehmomenterzeuger (213), welcher ausgestaltet ist, um ein Drehmoment zwischen der Oberschenkelverbindung und der Unterschenkverbindung zu erzeugen; und
einen Antrieb (215), welcher mit dem Drehmomenterzeuger gekoppelt ist,
ausgestaltet ist, um den Drehmomenterzeuger zu veranlassen, ein Drehmoment zu erzeugen, und ausgestaltet ist, um in einer Betriebsart zur Regenerierung von Leistung, in einer Betriebsart zur Verwendung von Leistung und in einer Betriebsart zur Abführung von Leistung zu arbeiten, wobei der Antrieb aufweist eine erste Hydraulikleitung zwischen einem Fluid-Vorratsbehälter (195) und dem hydraulischen Drehmomenterzeuger, wobei die erste Hydraulikleitung einen Hydraulik-Motor (217) umfasst, welcher mit einem den Motor isolierenden Ventil (216) in Reihe verbunden ist, wobei der Hydraulik-Motor drehbar mit einem elektrischen Generator (218) gekoppelt ist, und eine zweite Hydraulikleitung zwischen dem Fluid-Vorratsbehälter und dem hydraulischen Drehmomenterzeuger, welche ein gesteuertes Strömungsbeschränkungsventil (200) aufweist;
wobei, wenn sich der Antrieb in der Betriebsart zur Regenerierung von Leistung befindet, der Antrieb den Drehmomenterzeuger veranlasst, ein Drehmoment zu erzeugen, welches der Winkelgeschwindigkeit der Oberschenkelverbindung und
der Unterschenkverbindung relativ zueinander entgegenwirkt, und wobei der Antrieb einen Teil der Leistung, welche dem Produkt aus dem Drehmoment und
der Winkelgeschwindigkeit der Oberschenkelverbindung und der Unterschenkverbindung relativ zueinander entspricht, in eine elektrische Leistung wandelt, und wobei der Antrieb ausgestaltet ist, um in der Betriebsart zur Regenerierung von Leistung betrieben zu werden, wenn das Leistung erzeugende Bein auf einem ebenen Boden läuft;
wobei, wenn sich der Antrieb in der Betriebsart zur Regenerierung von Leistung befindet, sich das den Motor isolierende Ventil in einer offenen Stellung befindet und der Hydraulikmotor dem Hydraulikstrom, welcher durch die Bewegung des hydraulischen Drehmomenterzeugers verursacht wird, widersteht und ein elektrischer Strom durch die Anschlüsse des elektrischen Generators fließt, was anzeigt, dass eine elektrische Leistung durch den elektrischen Generator erzeugt wird;
wobei, wenn sich der Antrieb in der Betriebsart zur Verwendung von Leistung befindet, sich das den Motor isolierende Ventil in einer offenen Stellung befindet und der Hydraulikmotor durch den elektrischen Generator versorgt wird und den hydraulischen Drehmomenterzeuger antreibt, um ein Drehmoment zwischen der Oberschenkelverbindung und der Unterschenkverbindung zu erzeugen; und
wobei, wenn sich der Antrieb in der Betriebsart zur Abführung von Leistung befindet, sich das den Motor isolierende Ventil in einer geschlossenen Stellung befindet und das gesteuerte Strömungsbeschränkungsventil den Fluidstrom zwischen dem hydraulischen Drehmomenterzeuger und dem Fluid-Vorratsbehälter beschränkt und den hydraulischen Drehmomenterzeuger veranlasst, einer Bewegung der Oberschenkelverbindung und der Unterschenkverbindung zueinander zu widerstehen.

2. Leistung erzeugendes Bein nach Anspruch 1, **dadurch gekennzeichnet, dass**, wenn sich der Antrieb (215) in der Betriebsart zur Verwendung von Leistung befindet, der Antrieb, welcher gespeicherte elektrische Leistung einsetzt, den Drehmomenterzeuger (213) veranlasst, ein Drehmoment zu erzeugen.

3. Leistung erzeugendes Bein nach Anspruch 2, **dadurch gekennzeichnet, dass** der Antrieb ausgestaltet ist, um in der Betriebsart zur Verwendung von Leistung zu arbeiten, wenn sich das Leistung erzeugende Bein in einer Standphase befindet und eine Steigung und Stufen hinaufläuft.

4. Leistung erzeugendes Bein nach Anspruch 1, **dadurch gekennzeichnet, dass** die Oberschenkelverbindung (103) konfigurierbar ist, um mit einem Oberschenkel einer Person verbunden zu werden.

5. Leistung erzeugendes Bein nach Anspruch 1, **dadurch gekennzeichnet, dass** die Unterschenkelverbindung (105) konfigurierbar ist, um mit einem Unterschenkel einer Person verbunden zu werden.

6. Leistung erzeugendes Bein nach Anspruch 1, **dadurch gekennzeichnet, dass** die Oberschenkelverbindung (103) eine Fassung (254) umfasst, welche konfigurierbar ist, um an einem verbliebenen Oberschenkel eines Amputierten angebracht zu werden und daher das Leistung erzeugende Bein als eine Prothesevorrichtung oberhalb eines Knies fungiert.

7. Leistung erzeugendes Bein nach Anspruch 1, **dadurch gekennzeichnet, dass** der Antrieb (215) ausgestaltet ist, um in der Betriebsart zur Regenerierung von Leistung zu arbeiten, entweder:
wenn sich das Leistung erzeugende Bein in einer Standphase befindet; oder wenn sich das Leistung erzeugende Bein in einer Standphase befindet und gebeugt wird; oder
wenn sich das Leistung erzeugende Bein in einer Schwenkphase befindet.

8. Leistung erzeugendes Bein nach Anspruch 1, **dadurch gekennzeichnet, dass** der Antrieb (22) ausgestaltet ist, um in der Betriebsart zur Verwendung von Leistung zu arbeiten, wenn sich das Leistung erzeugende Bein in einer Standphase befindet und ausgestreckt wird.

9. Leistung erzeugendes Bein nach Anspruch 5, **dadurch gekennzeichnet, dass** das Leistung erzeugende Bein darüber hinaus einen künstlichen Fuß (256) umfasst, welcher mit der Unterschenkelverbindung (105) gekoppelt ist.

10. Leistung erzeugendes Bein nach Anspruch 1, **dadurch gekennzeichnet, dass** der Antrieb ausgestaltet ist, um in der Betriebsart zur Regenerierung von Leistung zu arbeiten, wenn sich das Leistung erzeugende Bein entweder a) in einer Standphase befindet und gebeugt wird oder b) in einer Standphase befindet und ein Gefälle und Stufen hinabläuft.

11. Leistung erzeugendes Bein nach Anspruch 1, **dadurch gekennzeichnet, dass** der Antrieb (215) darüber hinaus ein Einwegventil (199) umfasst, welches mit dem Drehmomenterzeuger (213) und dem Fluid-Vorratsbehälter (195) gekoppelt ist, wobei eine dritte Hydraulikleitung zwischen dem hydraulischen Drehmomenterzeuger und dem Fluid-Vorratsbehälter erzeugt wird, wobei für einen minimalen Widerstand für den hydraulischen Strom von dem Fluid-Vorratsbehälter zu dem Drehmomenterzeuger zu jeder Zeit gesorgt wird.

12. Leistung erzeugendes Bein nach Anspruch 1, darüber hinaus eine dritte Hydraulikleitung umfassend, welche einen zweiten elektrischen Generator (253) aufweist, welcher drehbar mit einem zweiten Hydraulikmotor (252) gekoppelt ist; wobei, wenn sich der Antrieb (215) in der Betriebsart zur Regenerierung von Leistung befindet, der erste und/oder der zweite Hydraulikmotor (217, 252) dem hydraulischen Strom widersteht, welcher durch die Bewegung des hydraulischen Drehmomenterzeugers (213) verursacht wird, und ein elektrischer Strom durch die Anschlüsse des entsprechenden ersten oder zweiten elektrischen Generators fließt, so dass eine elektrische Leistung durch den ersten oder zweiten elektrischen Generator erzeugt wird.

13. Leistung erzeugendes Bein nach Anspruch 12, **dadurch gekennzeichnet, dass** der Drehmomenterzeuger (213) mit dem Kniemechanismus gekoppelt ist; wobei der erste elektrische Generator (218) als ein elektrischer Motor in der Betriebsart zur Verwendung von Leistung eingesetzt wird, wenn sich das Leistung erzeugende Bein in einer Standphase befindet und eine Treppe oder eine Steigung hinaufläuft; wobei der erste elektrische Generator in der Betriebsart zur Regenerierung von Leistung eingesetzt wird, wenn sich das Leistung erzeugende Bein in einer Standphase befindet und eine Treppe oder ein Gefälle hinabläuft, und als ein elektrischer Motor in der Betriebsart zur Verwendung von Leistung eingesetzt wird, wenn sich das Leistung erzeugende Bein in einer Standphase befindet und eine Treppe oder Steigung hinaufläuft; und wobei der zweite elektrische Generator (253) in der Betriebsart zur Regenerierung von Leistung eingesetzt wird, wenn das Leistung erzeugende Bein auf einem ebenen Boden läuft.

14. Verfahren zum Betrieb eines Leistung erzeugenden Beins (10), welches ausgestaltet ist, um mit einem Unterschenkel einer Person gekoppelt zu werden, und aufweist: eine Oberschenkelverbindung (103); eine Unterschenkelverbindung (105); einen Kniemechanismus (107), welcher mit der Oberschenkelverbindung und der Unterschenkelverbindung verbunden ist und welcher ausgestaltet ist, um beugende und streckende Bewegungen der Oberschenkelverbindung und der Unterschenkelverbindung relativ zueinander zu ermöglichen; einen hydraulischen Drehmomenterzeuger (213), welcher ausgestaltet ist, um ein Drehmoment zwischen der Oberschenkelverbindung und der Unterschenkelverbindung zu erzeugen; und einen Antrieb (215) welcher mit dem Drehmomenterzeuger gekoppelt ist, ausgestaltet ist, um den Drehmomenterzeuger zu veranlassen, ein Drehmoment zu erzeugen, und ausgestaltet ist, um in einer Betriebsart zur Regenerierung von Leistung, in einer Betriebsart zur Verwendung von Leistung und in einer Betriebsart zur Abführung von Leistung zu arbeiten, wobei der Antrieb aufweist eine erste Hydraulikleitung zwischen einem Fluid-Vorratsbehälter (195) und dem hydraulischen Drehmomenterzeuger, wobei die erste Hydraulikleitung einen Hydraulikmotor (217) umfasst, welcher mit einem den Motor isolierenden Ventil in Reihe verbunden ist, wobei der Hydraulikmotor drehbar mit einem elektrischen Generator (218) gekoppelt ist, und eine zweite Hydraulikleitung zwischen dem Fluid-Vorratsbehälter und dem hydraulischen Drehmomenterzeuger, welche ein gesteuertes Strömungsbeschränkungsventil (200) aufweist; wobei das Verfahren umfasst:
wenn sich der Antrieb in der Betriebsart zur Regenerierung von Leistung befindet, veranlasst der Antrieb den Drehmomenterzeuger, ein Drehmoment zu erzeugen, welches der Winkelgeschwindigkeit der Oberschenkelverbindung und der Unterschenkelverbindung relativ zueinander entgegenwirkt und der Antrieb wandelt einen Teil der Leistung, welche dem Produkt aus den Drehmoment und der Winkelgeschwindigkeit der Oberschenkelverbindung und der Unterschenkelverbindung relativ zueinander entspricht, in eine elektrische Leistung, wobei der Antrieb in der Betriebsart zur Regenerierung von Leistung betreibbar ist, wenn das Leistung erzeugende Bein auf einem ebenen Boden läuft;
wenn sich der Antrieb in der Betriebsart zur Verwendung von Leistung befindet, sich das den Motor isolierende Ventil in einer offenen Stellung befindet und der Hydraulikmotor durch den elektrischen Generator angetrieben wird und der hydraulische Drehmomenterzeuger betätigt wird, um ein Drehmoment zwischen der Oberschenkelverbindung und der Unterschenkelverbindung zu erzeugen; und
wenn sich der Antrieb in der Betriebsart zur Abführung von Leistung befindet, sich das den Motor isolierende Ventil in einer geschlossenen Stellung befindet und das gesteuerte Strömungsbeschränkungsventil einen Fluid-Strom zwischen dem hydraulischen Drehmomenterzeuger und dem Fluid-Vorratsbehälter beschränkt und bewirkt, dass der hydraulische Drehmomenterzeuger einer Bewegung der Oberschenkelverbindung und der Unterschenkelverbindung zueinander widersteht; und
wobei, wenn sich der Antrieb in der Betriebsart zur Regenerierung von Leistung befindet, sich das den Motor isolierende Ventil in einer offenen Stellung befindet und der Hydraulikmotor dem hydraulischen Strom, welcher durch die Bewegung des hydraulischen Drehmomenterzeugers verursacht wird, widersteht und ein elektrischer Strom durch die Anschlüsse des elektrischen Generators fließt, was anzeigt, dass eine elektrische Leistung durch den elektrischen Generator erzeugt wird.

15. Verfahren nach Anspruch 14, **dadurch gekennzeichnet, dass** der Antrieb auch in der Betriebsart zur Regenerierung betreibbar ist:
wenn sich das Leistung erzeugende Bein in einer Standphase befindet; oder
wenn sich das Leistung erzeugende Bein in einer Standphase befindet und gebeugt wird; oder
wenn sich das Leistung erzeugende Bein in einer Schwenkphase befindet.

## Revendications

1. Jambe génératrice d'énergie (10), configurée pour être couplée à un membre inférieur d'une personne, comprenant :
une liaison de cuisse (103) ;
une liaison de jambe (105) ;
un mécanisme de genou (107) raccordé à ladite liaison de cuisse et à ladite liaison de jambe, et configuré pour permettre des mouvements de flexion et d'extension de ladite liaison de cuisse et de ladite liaison de jambe l'une par rapport à l'autre ;
un générateur de couple hydraulique (213) configuré pour générer un couple entre ladite liaison de jambe et ladite liaison de cuisse ; et
une unité d'énergie (215) couplée audit générateur de couple, configurée pour amener ledit générateur de couple à générer un couple et configurée pour fonctionner dans un mode de régénération d'énergie, un mode d'utilisation d'énergie et un mode de dissipation d'énergie, ladite unité d'énergie comprenant une première trajectoire hydraulique entre un réservoir de fluide (195) et le générateur de couple hydraulique, ladite première trajectoire hydraulique comprenant un moteur hydraulique (217) raccordé en série avec une soupape d'isolation de moteur (216), ledit moteur hydraulique étant couplé en rotation à un générateur électrique (218), et une seconde trajectoire hydraulique entre le réservoir de fluide et le générateur de couple hydraulique comprenant une soupape de limitation de débit (200) actionnée ;
dans laquelle, lorsque ladite unité d'énergie est dans le mode de régénération d'énergie, ladite unité d'énergie amène ledit générateur de couple à générer un couple qui oppose la vitesse angulaire de ladite liaison de cuisse et de ladite liaison de jambe l'une par rapport à l'autre, et ladite unité d'énergie transforme une partie de l'énergie associée au produit dudit couple et de ladite vitesse angulaire de ladite liaison de jambe et de la liaison de cuisse l'une par rapport à l'autre en énergie électrique, et dans laquelle ladite unité d'énergie est configurée pour fonctionner en mode de régénération d'énergie lorsque la jambe génératrice d'énergie marche sur un sol de niveau ;
dans laquelle lorsque ladite unité d'énergie est dans le mode de régénération d'énergie, ladite soupape d'isolation de moteur est dans une position ouverte et ledit moteur hydraulique résiste à l'écoulement hydraulique induit par le mouvement dudit générateur de couple hydraulique et un courant électrique passe par les bornes dudit générateur électrique indiquant que l'énergie électrique est générée par ledit générateur électrique ;
dans laquelle, lorsque ladite unité d'énergie est dans le mode d'utilisation d'énergie, ladite soupape d'isolation de moteur est dans une position ouverte et ledit moteur hydraulique est alimenté par le générateur électrique et actionne le générateur de couple hydraulique pour créer un couple entre la liaison de cuisse et la liaison de jambe ; et
dans laquelle, lorsque ladite unité d'énergie est dans le mode de dissipation d'énergie, ladite soupape d'isolation de moteur est dans une position fermée et ladite soupape de limitation de débit actionnée limite le débit de fluide entre le générateur de couple hydraulique et le réservoir de fluide et amène le générateur de couple hydraulique à résister au mouvement de la liaison de cuisse et de la liaison de jambe l'une par rapport à l'autre.

2. Jambe génératrice d'énergie selon la revendication 1, dans laquelle, lorsque ladite unité d'énergie (215) est dans le mode d'utilisation d'énergie, ladite unité d'énergie, utilisant l'énergie électrique stockée, amène le générateur de couple (213) à générer un couple.

3. Jambe génératrice d'énergie selon la revendication 2, dans laquelle ladite unité d'énergie est configurée pour fonctionner en mode d'utilisation d'énergie lorsque ladite jambe génératrice d'énergie est dans une phase de posture et monte des montées et des escaliers.

4. Jambe génératrice d'énergie selon la revendication 1, dans laquelle ladite liaison de cuisse (103) peut être configurée pour être raccordée à la cuisse d'une personne.

5. Jambe génératrice d'énergie selon la revendication 1, dans laquelle ladite liaison de jambe (105) peut être configurée pour être raccordée à la jambe d'une personne.

6. Jambe génératrice d'énergie selon la revendication 1, dans laquelle ladite liaison de cuisse (103) comprend une emboîture (254) pouvant être configurée pour être fixée à la cuisse restante d'une personne amputée et par conséquent, ladite jambe génératrice d'énergie fonctionne comme un dispositif de genou supérieur.

7. Jambe génératrice d'énergie selon la revendication 1, dans laquelle ladite unité d'énergie (215) est configurée pour fonctionner en mode de régénération d'énergie :
lorsque ladite jambe génératrice d'énergie est dans une phase de posture ; ou bien
lorsque ladite jambe génératrice d'énergie est dans une phase de posture et est en flexion ; ou bien
lorsque ladite jambe génératrice d'énergie est dans une phase d'oscillation.

8. Jambe génératrice d'énergie selon la revendication 1, dans laquelle ladite unité d'énergie (215) est configurée pour fonctionner en mode d'utilisation d'énergie lorsque ladite jambe génératrice d'énergie est dans une phase de posture et est en extension.

9. Jambe génératrice d'énergie selon la revendication 5, dans laquelle ladite jambe génératrice d'énergie comprend en outre un pied artificiel (256) couplé à ladite liaison de jambe (105).

10. Jambe génératrice d'énergie selon la revendication 1, dans laquelle ladite unité d'énergie est configurée pour fonctionner en mode de régénération d'énergie lorsque ladite jambe génératrice d'énergie est a) dans une phase de posture et est en flexion, ou bien b) dans une phase de posture et descend une descente et des escaliers.

11. Jambe génératrice d'énergie selon la revendication 1, dans laquelle ladite unité d'énergie (215) comprend en outre une soupape unidirectionnelle (199) couplée audit générateur de couple (213) et audit réservoir de fluide (195) créant une troisième trajectoire hydraulique entre ledit générateur de couple hydraulique et ledit réservoir de fluide fournissant un écoulement hydraulique de résistance minimum dudit réservoir de fluide audit générateur de couple à tous moments.

12. Jambe génératrice d'énergie selon la revendication 1, comprenant en outre une troisième trajectoire hydraulique comprenant un second générateur électrique (253) couplé en rotation à un second moteur hydraulique (252) ;
dans laquelle lorsque ladite unité d'énergie (215) est dans ledit mode de génération d'énergie, au moins l'un desdits premier ou second moteurs hydrauliques (217, 252) résiste à l'écoulement hydraulique induit par le mouvement dudit générateur de couple hydraulique (213) et un courant électrique passe par les bornes du premier ou second générateur électrique correspondant de sorte que l'énergie électrique est générée par ledit premier ou second générateur électrique.

13. Jambe génératrice d'énergie selon la revendication 12, dans laquelle le générateur de couple (213) est couplé au mécanisme de genou ; ledit premier générateur électrique (218) est utilisé en tant que moteur électrique en mode d'utilisation d'énergie lorsque ladite jambe génératrice d'énergie est dans une phase de posture et monte un escalier ou une montée ; ledit premier générateur électrique est utilisé en mode de régénération d'énergie lorsque ladite jambe génératrice d'énergie est dans une phase de posture et descend un escalier ou une descente et est utilisée en tant que moteur électrique en mode d'utilisation d'énergie lorsque ladite jambe génératrice d'énergie est dans une phase de posture et monte un escalier ou une montée ; et ledit second générateur électrique (253) est utilisé en mode de régénération d'énergie lorsque ladite jambe génératrice d'énergie marche sur un sol de niveau.

14. Procédé pour actionner une jambe génératrice d'énergie (10) configurée pour être couplée à un membre inférieur d'une personne et comprenant : une liaison de cuisse (103) ; une liaison de jambe (105) ; un mécanisme de genou (107) raccordé à ladite liaison de cuisse et ladite liaison de jambe, et configuré pour permettre des mouvements de flexion et d'extension de ladite liaison de cuisse et de ladite liaison de jambe l'une par rapport à l'autre ; un générateur de couple hydraulique (213) configuré pour générer un couple entre ladite liaison de jambe et ladite liaison de cuisse ; et une unité d'énergie (215) couplée audit générateur de couple, configurée pour amener ledit générateur de couple à générer un couple et configurée pour fonctionner en mode de régénération d'énergie, mode d'utilisation d'énergie et mode de dissipation d'énergie, ladite unité d'énergie comprenant une première trajectoire hydraulique entre un réservoir de fluide (195) et le générateur de couple hydraulique, ladite première trajectoire hydraulique comprenant un moteur hydraulique (217) raccordé en série avec une soupape d'isolation de moteur (216), ledit moteur hydraulique étant couplé en rotation à un générateur électrique (218), et une deuxième trajectoire hydraulique entre le réservoir de fluide et le générateur de couple hydraulique comprenant une soupape de limitation de débit actionnée (200), ledit procédé comprenant :
lorsque ladite unité d'énergie est en mode de régénération d'énergie, ladite unité d'énergie amenant ledit générateur de couple à générer un couple qui s'oppose à la vitesse angulaire de ladite liaison de cuisse et ladite liaison de jambe l'une par rapport à l'autre et ladite unité d'énergie transformant une partie de l'énergie associée au produit dudit couple et de ladite vitesse angulaire de ladite liaison de jambe et la liaison de cuisse l'une par rapport à l'autre en énergie électrique, ladite unité d'énergie pouvant fonctionner en mode de régénération d'énergie lorsque la jambe génératrice d'énergie marche sur un sol de niveau ;
lorsque ladite unité d'énergie est en mode d'utilisation d'énergie, ladite soupape d'isolation de moteur étant dans une position ouverte et ledit moteur hydraulique étant alimenté par le générateur électrique et actionnant le générateur de couple hydraulique pour créer un couple entre la liaison de cuisse et la liaison de jambe ; et
lorsque l'unité d'énergie est en mode de dissipation d'énergie, ladite soupape d'isolation de moteur étant dans une position fermée et ladite soupape de limitation de débit actionnée limitant le débit entre le générateur de couple hydraulique et le réservoir de fluide et amenant le générateur de couple hydraulique à résister au mouvement de la liaison de cuisse et de la liaison de jambe l'une par rapport à l'autre ; et
dans lequel lorsque ladite unité d'énergie est en mode de régénération d'énergie, ladite soupape d'isolation de moteur est dans une position ouverte et ledit moteur hydraulique résiste à l'écoulement hydraulique induit par le mouvement dudit générateur de couple hydraulique et un courant électrique passe par les bornes dudit générateur électrique indiquant que l'énergie électrique est générée par ledit générateur électrique.

15. Procédé selon la revendication 14, dans lequel ladite unité d'énergie peut également fonctionner en mode de régénération ;
lorsque ladite jambe génératrice d'énergie est dans une phase de posture ; ou bien
lorsque ladite jambe génératrice d'énergie est dans une phase de posture et est en flexion ; ou bien
lorsque ladite jambe génératrice d'énergie est dans une phase d'oscillation.
